# EUROPÄISCHE PATENTSCHRIFT

(11) **EP 2 050 384 B1**
(45) Veröffentlichungstag und Bekanntmachung des Hinweises auf die Patenterteilung: **11.08.2010**
(21) Anmeldenummer: 07405308.3
(22) Anmeldetag: 16.10.2007
(51) Int. Cl.: A61B 5/00, G01D 1/16

(54) **Verfahren zur Speicherung einer Messreihe**
Method for storing a measurement data series
Procédé destiné au stockage d'une rangée de mesure

(43) Veröffentlichungstag der Anmeldung: 22.04.2009
(73) Patentinhaber: Roche Diagnostics GmbH, 68305 Mannheim (DE); F.HOFFMANN-LA ROCHE AG, 4070 Basel (CH)
(72) Erfinder: Krieftewirth, Michael, 3429 Ersigen (CH)
(74) Vertreter: Rüfenacht, Philipp Michael

(56) Entgegenhaltungen:
- WO-A-2005/065542
- WO-A-2007/116226
- US-A1- 2005 038 332

## Beschreibung

### Technisches Gebiet

Die Erfindung betrifft ein Verfahren zum Speichern einer Reihe von gemessenen Werten, die einen zeitabhängigen in oder am menschlichen Körper gemessenen Parameter repräsentieren, insbesondere eine physiologische Glukosekonzentration. Die Erfindung betrifft weiter eine Vorrichtung sowie ein Computerprogrammprodukt zur Durchführung des Verfahrens.

### Stand der Technik

Der Körper eines Menschen, der an Diabetes leidet, ist nicht dazu in der Lage, eine ausreichende Menge Insulin zu produzieren, oder er reagiert nicht angemessen auf das von ihm produzierte Insulin. Diese Tatsache führt zu einem Ungleichgewicht der Glucosekonzentration im Blut (damit zu Hyper- oder Hypoglycämien), was die Ursache für schwerwiegende Konsequenzen, wie Ketoazidose, Komplikationen der Blutgefässe, Krämpfe oder Bewusstlosigkeit sein kann. Um einen gesunden Blutzuckerspiegel aufrecht zu erhalten, befolgen Diabetespatienten gewöhnlich strenge Ernährungsprogramme und verbinden diese mit einer Basalinsulinabgabe und selektiven Insulinboli. Die Insulinabgabe muss dabei individuell auf den Körper des Patienten abgestimmt sein, um diesen zur richtigen Zeit mit der richtigen Menge von Insulin zu versorgen. Um Zeitpunkt und Menge eines nächsten Insulinbolus zu bestimmen, messen Patienten regelmässig die Glucosekonzentration ihres Blutes und bestimmen den Kohlehydratgehalt ihrer Mahlzeiten.

Anstatt Blutzuckermessungen mit Streifenmessgeräten vorzunehmen, was je nach Intensität der durchgeführten Therapie drei bis sechs und nur in Ausnahmefällen zehn oder mehr Male pro Tag geschieht, besteht die Möglichkeit, kontinuierlich arbeitende Blutzuckermessgeräte zu verwenden. Einer der Vorzüge eines kontinuierlich arbeitenden Messsystems liegt in der Möglichkeit zur Berechnung von Trendinformationen, welche erst bei einer hohen Rate an Messdaten sinnvoll durchgeführt werden kann. Die berechneten Trendinformationen beziehen sich dabei zumeist auf die zuvor gemessenen Werte und geben somit Informationen über den Verlauf der Glucosekonzentration in der nahen Zukunft. Äussere Einflüsse wie die Verabreichung eines Insulinbolus, eine Einnahme von Mahlzeiten oder sportliche Aktivitäten beeinflussen die Genauigkeit der Trendinformation. Eine Speisung eines Messsystems mit Informationen über diese äusseren Einflüsse kann die Genauigkeit der Trendberechnung deutlich steigern und ermöglicht damit insbesondere auch, die Dosierung eines Insulinbolus genauer an die Bedürfnisse eines Patienten anzupassen.

Blutzuckermessgeräte zur kontinuierlichen Erhebung von Glucosedaten sind in der Regel tragbar. Datenspeicher zur Speicherung der erfassten Daten machen bei derartigen Geräten einen erheblichen Anteil der Gerätekosten aus. Bei derzeit üblichen Abtast-Intervallen ("Sampling") von z. B. 5 Min. entsteht bereits eine grosse Datenmenge, welche mit herkömmlicher Datenkompression nicht ohne Verlust wertvoller Informationen effektiv komprimiert werden kann. Zudem ist beispielsweise eine retrospektive Trendberechnung mit ausreichender Genauigkeit für die weitere Datenauswertung selbst bei 5 minütigen Speicherintervallen nur schwer möglich, insbesondere, wenn das gespeicherte Signal zusätzlich von einem Rauschen überlagert ist.

Glukosemessungen, wobei Daten aufgezeichnet und gespeichert werden, sind aus WO 2005/065542 A2 bekannt.

### Darstellung der Erfindung

Aufgabe der Erfindung ist es daher, ein dem eingangs genannten technischen Gebiet zugehörendes Verfahren zu schaffen, welches eine Reduktion der gespeicherten Datenmenge ermöglicht, wobei eine retrospektive Rekonstruktion der gemessenen Werte mit ausreichender Genauigkeit möglich ist.

Die Lösung der Aufgabe ist durch die Merkmale des Anspruchs 1 definiert. Gemäss der Erfindung wird ein Verfahren zum Speichern einer Reihe von gemessenen Werten bereitgestellt, die einen zeitabhängigen in oder am menschlichen Körper gemessenen Parameter repräsentieren. Erfindungsgemäss umfasst die Reihe eine erste Anzahl von gemessenen Werten, wobei eine zweite Anzahl von Werten gespeichert wird und dabei die zweite Anzahl geringer ist als die erste Anzahl.

Hierbei können die gemessenen Parameter bereits interpretierte bzw. ermittelte oder abgeleitete Werte sein, z. B. Absolutwerte der Glukosekonzentration, aber auch teilverarbeitete Daten und/oder Rohdaten wie z. B. Spannungs- oder Stromwerte, welche direkt von entsprechenden Mess-Sensoren ausgegeben werden. Im Weiteren wird hier und im Folgenden unter einer "kontinuierlich" ermittelten Messreihe eine Messdaten-Aquirierung verstanden, welche quasikontinuierlich mit einer gewissen, insbesondere im Vergleich mit der Variabilität des tatsächlichen zeitlichen Verlaufs des gemessenen Parameters kurzen, Messperiode erfolgt. Die Messperiode ist derart klein gewählt, dass davon ausgegangen werden kann, dass der tatsächliche Verlauf des gemessenen Parameters durch die Messreihe weitgehend wiedergegeben wird. Typische Messperioden betragen einige Sekunden bis einige Minuten. "Kontinuierliche" Messungen stehen hierbei insbesondere im Gegensatz zu bekannten Blutzuckermessungen mit beispielsweise Streifenmessgeräten, welche je nach Intensität der durchgeführten Therapie drei bis sechs und nur in Ausnahmefällen zehn oder mehr Male pro Tag durchgeführt werden.

Dem erfindungsgemässen Verfahren liegt der Gedanke zugrunde, dass nicht jeder gemessene Wert der Messreihe dieselbe Relevanz für eine spätere, retrospektive Rekonstruktion der Messreihe mit hinreichender Genauigkeit hat. Um den zeitlichen Verlauf der Messreihe rekonstruieren zu können, ist es gemäss der Erfindung demnach ausreichend, eine geringere Anzahl von Werten abzuspeichern als gemessen wurde. Messwerte, die aufgrund vorgegebener und/oder aus den Messdaten zu bestimmender Kriterien als weniger relevant bzw. als irrelevant für den Verlauf der Messkurve beurteilt werden, werden ausgesondert und nicht abgespeichert. Durch eine derartige unvollständige bzw. teilweise Speicherung von Messwerten der kontinuierlichen Messreihe ist die Anzahl von gespeicherten Werten kleiner als die Anzahl von gemessenen Werten und die gespeicherte Gesamt-Datenmenge der Messreihe (d. h. der kontinuierlich erfassten Glucosewerte) ist somit reduziert. Es kann dabei durchaus auch vorteilhaft sein, zusätzliche, beispielsweise von den Messwerten abgeleitete, Werte zu speichern, wobei aber gemäss der Erfindung auch in diesem Fall die Anzahl der gesamthaft zu speichernden Werte geringer als die Anzahl der gemessenen Werte ist.

Ob ein Messpunkt relevant für den Kurvenverlauf ist und damit gespeichert werden soll, kann aufgrund eines dem Messwert zugeordneten Speicherkriteriums ermittelt werden. Mögliche Speicherkriterien können beispielsweise fest vorgegeben sein und auf einer *a priori* bekannten zeitlichen Variabilität der Messkurve basieren. Aufgrund der vorbekannten Variabilität kann z. B. festgestellt werden, mit welchem Zeitabstand bzw. mit welchem Speicherintervall gemessene Werte gespeichert werden müssen, damit eine retrospektive Reproduktion der Messkurve mit hinreichender Genauigkeit gewährleistet ist. Je nach Messperiode ist es beispielsweise hinreichend, nur jeden n-ten Messwert abzuspeichern, wobei n beispielsweise auch von der Variabilität der gemessenen Werte abhängt.

Andere Speicherkriterien beruhen auf Analysen der gemessenen Werte bzw. davon abgeleiteten Werten und/oder auf weiteren, zusätzlichen ermittelten Daten und ergeben beispielsweise variable Speicherintervalle. Derartige Speicherkriterien werden mit Vorteil aufgrund der gemessenen Werte und/oder weiterer ermittelter Parameter laufend angepasst bzw. für beispielsweise jeden neuen Messwert erneut berechnet und ausgewertet.

Eine Möglichkeit für ein adaptives Speicherkriterium eines Messwertes umfasst beispielsweise die Ermittlung einer Abweichung bzw. eines Fehlerwerts zuletzt gemessener Werte von einer Vorhersage bzw. Extrapolation aus den gespeicherten Werten. Übersteigt die Abweichung ein gewisses Mass, so kann daraus geschlossen werden, dass eine Abspeicherung eines weiteren Messwerts erforderlich ist. Dabei kann z. B. nur ein zuletzt gemessener Wert mit der Vorhersage verglichen werden oder aber auch eine Reihe von aktuellen Messwerten. Auswahlkriterien können insbesondere auch aus mehreren, dem tragbaren Gerät zur Verfügung stehenden, Messwerten der Messreihe hergeleitet werden. Eine kurze Zwischenspeicherung in beispielsweise einem Puffer- bzw. Zwischenspeicher stellt dabei den Zugriff auf die kontinuierlichen Messwerte in einem vorgebbaren oder den Erfordernissen angepassten Zeitfenster einer gewissen Zeitdauer sicher. Insbesondere können sämtliche Messwerte im Zwischenpuffer zur Filterung und/oder auch zur ausreichend genauen Berechnung von z. B. Trends (z. B. erste zeitliche Ableitung der Messwerte) oder anderen messreihenverlaufs-bezogenen Parametern (z. B. höhere zeitliche Ableitungen, Varianz der Messdaten in einem Zeitfenster, Extrapolation etc.) herangezogen werden.

Ein Speicherintervall bzw. ein Speicherkriterium kann bevorzugt auch derart gewählt bzw. angepasst werden, dass Fluktuationen einer gewissen zeitlichen Skala ausgefiltert werden können (siehe hierzu z. B. Nyquist-Kriterium). Insbesondere kann ein Speicherintervall so angepasst werden, dass zwar der komplette physiologische Kurvenverlauf retrospektiv im Wesentlichen wiederhergestellt werden kann, ein Rauschen und/oder andere gerätebedingte kurzzeitige Messungenauigkeiten aber herausgefiltert sind. Mit einer derartigen Auswahl der zu speichernden Messwerte erfolgt eine Filterung im Sinne eines Tiefpassfilters, und ergibt eine Glättung einer später rekonstruierten Messkurve.

Schliesslich können zur Ermittlung eines Speicherintervalls bzw. Speicherkriteriums beispielsweise auch zusätzliche Informationen zu Ereignissen herangezogen werden, welche den gemessenen Parameter beeinflussen. Beispielsweise können für den Fall, dass der gemessene Parameter eine Glucosekonzentration im Blut eines Menschen ist, Art, Zeitpunkt oder beispielsweise der Kohlehydratgehalt eingenommener Mahlzeiten, verabreichte Insulinboli oder die Art und Dauer einer betriebenen sportlichen Aktivität mit einbezogen werden.

Um eine bessere Anpassung einer aus den gespeicherten Werten rekonstruierbaren Messkurve an den Verlauf der kontinuierlichen Messreihe zu gewährleisten, ist in einer bevorzugten Variante des Verfahrens ein erster Speichermodus vorgesehen, in welchem ein zeitlicher Abstand zwischen aufeinander folgenden gespeicherten Werten variabel ist. Damit wird erreicht, dass Speicherintervalle der gemessenen Werte, d. h. die Zeitabstände zwischen zwei gespeicherten Werten, den Erfordernissen angepasst werden können.

Varianten des Verfahrens umfassen beispielsweise unterschiedliche aber z. B. in Abhängigkeit einer Tageszeit oder spezieller Phasen fest vorgegebene Speicherintervalle. Dabei können in Zeiträumen geringer Dynamik wie z. B. in einem Fastenzustand oder in der Nacht längere Speicherintervalle vorgegeben werden, wohingegen in Tagesphasen höherer Dynamik kürzere Speicherintervalle vorgegeben sein können. Vorzugsweise sind die Speicherintervalle derart fest vorgegeben, dass die ursprünglich gemessenen Messwerte durch die gespeicherten Werte mit genügend grosser Genauigkeit wiedergeben werden können.

Bevorzugt wird jedoch im ersten Speichermodus der zeitliche Abstand zwischen den gespeicherten Werten in Abhängigkeit einer zeitlichen Variabilität der gemessenen Werte angepasst. Das Verfahren ist dabei derart adaptiv, dass beispielsweise, insbesondere automatisch, in Zeiträumen mit geringer Dynamik vergleichsweise selten ein gemessener Wert gespeichert wird und in Zeiträumen hoher Dynamik gemessene Werte vergleichsweise häufig abgespeichert werden. Bevorzugt wird eine Variabilitätsanalyse aufgrund der im Zwischenpuffer zwischengespeicherten kontinuierlichen Messwerte durchgeführt. Mit der Variabilitätsanalyse kann z. B. ein Zeitpunkt für die nächste Speicherung eines Messwertes festgelegt werden. Aufgrund der Variabilitätsanalyse kann aber auch ein Speicherkriterium ermittelt werden, welches beispielsweise einem bereits gemessenen Wert im Zwischenpuffer, z. B. einem zuletzt gemessenen Wert, zugeordnet wird und dessen Auswertung über eine Speicherung des zugeordneten Wertes entscheidet. Aufgrund einer auf diese Weise adaptiven Anpassung der Speicherintervalle kann eine für eine hinreichend gute Rekonstruktion der Kurve erforderliche Datenmenge weiter reduziert werden.

In einer besonders bevorzugten Variante des erfindungsgemässen Verfahrens wird aus einer Interpolation zwischen einem letzten gespeicherten Wert und einem zuletzt gemessenen Wert in Bezug auf einen entsprechenden Bereich der Reihe von gemessenen Werten ein Fehlermass gebildet. Dabei wird ein gemessener Wert des entsprechenden Bereichs der Reihe gespeichert, wenn das Fehlermass einen gewissen Maximalwert überschreitet. Das Fehlermass ergibt somit ein Speicherkriterium für einen gemessenen Wert im entsprechenden Bereich der Reihe, d. h. im Bereich der Reihe zwischen einem zuletzt gespeicherten Wert und einem zuletzt gemessenen Wert. Insbesondere umfasst dabei der interpolierte Bereich auch einen zuletzt gemessenen Wert, welcher in einer möglichen Variante des Verfahrens aufgrund des Überschreitens eines Maximalwerts des Fehlermasses gespeichert wird.

Mit anderen Worten wird der zeitliche Verlauf des gemessenen Parameters in einem zeitlichen Abschnitt zwischen dem Zeitpunkt eines zuletzt gespeicherten und dem Zeitpunkt eines zuletzt gemessenen Werts interpoliert. Die im Puffer zwischengespeicherten kontinuierlichen Werte in diesem Zeitabschnitt werden mit der Interpolation verglichen und aus dem Abstand des interpolierten Verlaufs und dem tatsächlichen Verlauf wird ein Fehlermass bestimmt. Dabei kann z. B. die (normierte) grösste Abweichung der beiden Kurven oder eine (normierte) Fläche zwischen den beiden Kurven im gegebenen Zeitabschnitt als Fehlermass herangezogen werden. Grundsätzlich sind hierfür aber auch sämtliche beispielsweise aus der Masstheorie bekannten Masse geeignet, welche auf irgendeine Art einen Abstand zweier Kurven in einem vorgegebenen Bereich zu quantifizieren vermögen.

Übersteigt das Fehlermass einen gewissen vorgebbaren oder fest vorgegebenen Toleranzwert bzw. Maximalwert, so gilt das Speicherkriterium des zuletzt gemessenen Werts als erfüllt. Mit anderen Worten kann durch das Fehlermass festgestellt werden, ob ein neuer Messwert gespeichert werden muss, damit bei einer späteren Rekonstruktion der Messkurve eine vorgegebene Toleranz bzw. Abweichung nicht überschritten wird. Je nach Variante des erfindungsgemässen Verfahrens kann es aber auch vorteilhaft sein, nicht einen zuletzt gemessenen Wert, sondern beispielsweise einen vor dem letzten Wert gemessenen Wert im Zwischenpuffer, insbesondere einen als vorletzten gemessenen Wert, beim Überschreiten des Maximalwerts zu speichern, da bei diesem Messwert das Fehlermass noch nicht überschritten war.

Aufgrund der Interpolation kann somit erfindungsgemäss eine Vorhersage des Messwertverlaufs aufgrund früherer gespeicherter Werte und/oder in einem Zwischenpuffer abgelegter kontinuierlicher Messwerte in die Auswahl der zu speichernden Werte mit einbezogen werden. Die Interpolation kann beispielsweise mit einer Gewichtung der im Puffer abgelegten Messwerte beeinflusst werden, indem beispielsweise älteren Werten ein geringeres Gewicht zugeordnet wird, als jüngeren und aktuelleren Werten.

In einer bevorzugten Variante des erfindungsgemässen Verfahrens erfolgt die Interpolation als eine lineare Interpolation des Zeitabschnitts zwischen dem Zeitpunkt eines letzten gespeicherten Werts und dem Zeitpunkt eines zuletzt gemessenen Werts. Ein lineares Interpolationsverfahren erfordert einen nur geringen Rechenaufwand und erzielt damit eine hohe Geschwindigkeit. Somit müssen nur vergleichsweise geringe Ressourcen für die Interpolation bereitgestellt werden. Zur Bestimmung des Fehlermasses als Basiskriterium für eine Auswahl von Messwerten zur Speicherung ist die mit linearer Interpolation erzielte Genauigkeit für die meisten Anforderungen hinreichend. Es können jedoch durchaus auch Varianten des Verfahrens bevorzugt sein, welche sich Interpolationsverfahren höherer Ordnung bedienen, um eine bessere Genauigkeit zu erzielen. Wie das Interpolationsverfahren gewählt wird, hängt im Wesentlichen davon ab, wie das Fehlermass bzw. dessen Maximalwert definiert ist und wie die Messkurve aus den gespeicherten Werten später rekonstruiert wird. Aufgrund des geringeren Aufwands an benötigten Ressourcen und der recht guten Genauigkeit ist jedoch ein lineares Verfahren, sofern anwendbar, bevorzugt.

Um die Anpassung des Speicherintervalls weiter optimieren zu können ist bei einer bevorzugten Variante des erfindungsgemässen Verfahrens ein zweiter Speichermodus vorgesehen, in welchem der zeitliche Abstand zwischen aufeinander folgenden gespeicherten Werten konstant ist. Dabei kommt insbesondere der erste Speichermodus zur Anwendung, wenn eine Variabilität der gemessenen Werte einen vorgegebenen Wert unterschreitet und der zweite Speichermodus kommt zur Anwendung, wenn die Variabilität der gemessenen Werte den vorgegebenen Wert überschreitet.

Bei hoher Variabilität kann eine laufende Analyse und adaptive Anpassung der Speicherintervalle zu einem hohen Rechenaufwand führen, welcher unerwünscht sein kann. Es ist in diesem Fall daher mitunter von Vorteil, das Speicherintervall konstant zu halten und auf eine laufende Analyse vor jeder Speicherung eines Wertes zu verzichten. Die Speicherintervalle sind dabei bevorzugt hinreichend kurz festzulegen, um ein gutes Sampling der Messkurve durch die gespeicherten Werte sicherzustellen. Die Variabilität der Messkurve kann dabei im ersten Speichermodus z. B. sporadisch analysiert werden, um gegebenenfalls z. B. im Fall einer geringeren Variabilität automatisch in den zweiten Speichermodus umzuschalten. Im zweiten Speichermodus können zeitliche Abstände zwischen zwei aufeinander folgenden gespeicherten Werten auf Zeiträume ausgedehnt werden, in welchen die geringe Variabilität kein neuerliches Speichern eines Messwertes erfordert. Eine Umschaltung zwischen den beiden Speichermodi erfolgt bevorzugt automatisch aufgrund einer Variabilitätsanalyse, kann aber auch manuell erfolgen, wenn beispielsweise zu einer unerwarteten Tageszeit eine erhöhte Dynamik zu erwarten ist (z. B. unerwartete Nachtaktivität)

Varianten des Verfahrens, welche einen Betrieb in nur einem der beiden Speichermodi zulassen, können je nach Anforderung bevorzugt sein. Mit Blick auf eine grösstmögliche Flexibilität in der Anpassung des Speicherverfahrens an einen Verlauf der Messkurve ist jedoch ein Verfahren, bei welchem beide Speichermodi vorgesehen sind, zu bevorzugen.

Das erfindungsgemässe Verfahren ist aber nicht auf eine Speicherung bzw. Analyse von gemessenen Werten beschränkt. Um zusätzliche Informationen zum Verlauf der Messkurve zu gewinnen, können auch abgeleitete Grössen aus den Messwerten ermittelt werden. Insbesondere können aus zuvor gemessenen Werten beispielsweise Trendinformationen gewonnen werden, welche eine Schätzung des weiteren Verlaufs der Messkurve, d. h. der Glucosekonzentration in der nahen Zukunft, geben. In einer bevorzugten Variante des erfindungsgemässen Verfahrens werden daher aus der Reihe der gemessenen Werte Trendwerte ermittelt. Mögliche Trendwerte umfassen dabei z. B. eine erste Ableitung nach der Zeit der gemessenen Werte.

Aufgrund der ermittelten Trendwerte kann beispielsweise die Vorhersagekraft des weiteren Messkurvenverlaufs aufgrund zwischengespeicherter Messwerte deutlich verbessert bzw. vereinfacht werden. Insbesondere kann durch Beiziehen von Trendinformationen eine Vorhersage bei der Interpolation zur Bestimmung des Fehlermasses verbessert bzw. vereinfacht werden.

Bevorzugt wird zur Ermittlung der Trendwerte bzw. des Fehlermasses ein Kalmän-Filter eingesetzt, ein bekannter Zustandsschätzer für zeitdiskrete Systeme. Das Kalmän-Filter wird insbesondere dafür verwendet, Zustände oder Parameter des Systems aufgrund von teils redundanten Messungen, die von Rauschen überlagert sind, zu schätzen. Dabei wird der mittlere quadratische Fehler minimiert. Beim Kalmán-Filter handelt es sich um einen adaptiven Filter, wodurch die entsprechenden Werte laufend verbessert werden und stets eine gute Schätzung für den Trend bzw. für das Fehlermass vorliegt.

Um eine spätere Rekonstruktion der Messkurve aus den gespeicherten Daten zu vereinfachen bzw. zu verbessern und/oder insbesondere die zu speichernde Datenmenge bei gleich bleibender Reproduzierbarkeit der Messkurve zu erhalten, werden vorzugsweise neben den gespeicherten Werten auch Trendwerte gespeichert. Die Trendwerte können beispielsweise mit den ausgewählten Werten als gleichzeitiges Wertepaar gespeichert werden. Die Speicherung von Trendwerten kann aber auch im Speicherintervall zwischen der Speicherung zweier aufeinander folgender Werte erfolgen. Insbesondere ist bei grossen Speicherintervallen der gespeicherten Messwerte eine Trendbestimmung schwierig, weshalb eine zusätzliche Speicherung der Trendwerte die Rekonstruierbarkeit der Messkurve aufgrund der gespeicherten Werte verbessert.

In dieser Variante des Verfahrens werden neben den gemessenen Werten zwar weitere Werte gespeichert. Durch die zusätzliche Trendinformation kann aber die nächste Speicherung erst nach einem vergleichsweise langen Zeitraum erforderlich werden, wodurch gesamthaft die zu speichernde Datenmenge zur Rekonstruktion der Messkurve mit einer vorgegebenen Qualität weiter reduziert werden kann.

In einer bevorzugten Variante des Verfahrens wird der aktuell gemessene Wert gespeichert, wenn eine dem aktuell gemessenen Wert zugeordnete Änderung des Trendwerts einen vorgegebenen Minimalwert überschreitet. Insbesondere wird ein gemessener Wert genau dann gespeichert, wenn ein Betrag der ersten zeitlichen Ableitung der Trendwerte einen gewissen Minimalwert überschreitet. Bevorzugt wird hierbei bei einer betragsmässigen Überschreitung des Minimalwertes für die Trendänderung ein Wertepaar abgespeichert, welches den entsprechenden Messwert sowie einen zugehörigen Trendwert umfasst.

Die Ableitung kann dabei zum Zeitpunkt der Messung des zu speichernden Wertes ermittelt werden, oder zu einem anderen Zeitpunkt und dem zu speichernden Wert zugeordnet werden. Es ist z. B. denkbar, dass der Betrag einer zeitlichen Ableitung der Trendwerte zum Zeitpunkt eines zuletzt gespeicherten Wertes ein Speicherkriterium eines aktuellen Messwertes bildet.

Bevorzugt werden aus der Reihe von gemessenen Werten Trendänderungswerte ermittelt, die einer zweiten zeitlichen Ableitung der gemessenen Werte entsprechen. Dies ist insbesondere dann der Fall, wenn für die Trendwerte die erste zeitliche Ableitung der gemessenen Werte herangezogen wird. Mit anderen Worten wird in diesem Fall ein gemessener Wert dann abgespeichert, wenn der Betrag einer ihm zugeordneten zweiten zeitlichen Ableitung einen vorgegebenen Minimalwert überschreitet. Die Trendänderungswerte können lediglich zur Bestimmung des Speicherkriteriums genutzt werden oder zusätzlich zu den Messwerten und/oder Trendwerten abgespeichert werden.

Vorzugsweise sind in einer weiteren Variante des erfindungsgemässen Verfahrens zeitliche Stützstellen vorgesehen, an welchen der entsprechende Trendwert und/oder Trendänderungswert, nicht aber der gemessene Wert selbst abgespeichert wird. Es ist z. B. denkbar, dass der Trendwert bzw. der Trendänderungswert im Zeitintervall zwischen zwei gespeicherte Werte gespeichert wird. Dabei kann der Zeitpunkt der Speicherung der Trendwerte bzw. ein Trendänderungswert adaptiv aufgrund der Variabilität der Messkurve bestimmt werden oder beispielsweise in Bezug auf einen Zeitraum zwischen zwei aufeinander folgenden gespeicherten Werten fest vorgegeben sein, z. B. in der zeitlichen Mitte.

Mit der zusätzlichen Ermittlung und gegebenenfalls der Speicherung von Trendwerten bzw. Trendänderungswerten zu den ausgewählten gemessenen Werten wird erreicht, dass das erfindungsgemässe Speicherverfahren auf vorteilhafte Weise eine optimale Anpassung an eine variable Messkurve erlaubt, um die für eine hinreichend genaue Rekonstruktion der Messkurve notwendige Datenmenge zu verringern bzw. bestenfalls zu minimieren.

Eine Vorrichtung zur Durchführung eines Verfahrens, das wie oben beschrieben werden kann, umfasst eine Messvorrichtung zum kontinuierlichen Messen einer Reihe von gemessenen Werten eines an oder in einem menschlichen Körper gemessenen Parameters, insbesondere einer Glucosekonzentration. Weiter umfasst die Vorrichtung eine Systemsteuerung mit einer Speichereinheit zum Speichern der Messwerte, wobei die Systemsteuerung derart ausgebildet und programmiert ist, dass bei einer ersten Anzahl der von der Reihe umfassten Werte eine zweite Anzahl von Werten speicherbar ist, wobei die zweiten Anzahl kleiner ist als die erste Anzahl.

Die erfindungsgemässe Vorrichtung umfasst mit Vorteil alle vorgenannten Elemente, um als funktionale Einheit arbeiten zu können. Zumindest die Systemsteuerung muss entsprechende Ein- und Ausgabevorrichtungen aufweisen oder adressieren können. Es ist nicht zwingend erforderlich, dass eine Messvorrichtung in die erfindungsgemässe Vorrichtung integriert ist. Die Messwerte sind der Vorrichtung aber auf gewisse Weise zuzuführen bzw. müssen in diese eingespiesen werden, was beispielsweise auch über eine externe Messvorrichtung geschehen kann. Analoges gilt für die Ausgabe der Daten, welche sich zwar bevorzugt einer beispielsweise in der erfindungsgemässen Vorrichtung enthaltenen Anzeige bedient. Es ist aber im Prinzip ausreichend, dass eine Vorrichtung zum Übertragen von Daten an ein insbesondere externes Anzeigegerät vorhanden ist.

Bevorzugt ist die gesamte Vorrichtung jedoch in ein einzelnes Gerät integriert, welches mit Vorteil tragbar ausgestaltet ist. Insbesondere ist das tragbare Gerät derart ausgestaltet, dass ein Benutzer durch das Gerät nicht oder nur unwesentlich in seiner Bewegungsfreiheit eingeschränkt ist. Zum Datenauslesen bzw. zum Laden einer Batterie kann das Gerät beispielsweise an einen PC oder eine andere Basisstation angeschlossen werden. Das tragbare Gerät kann zudem Anzeigemittel wie ein Display sowie Steuerelemente umfassen, um einerseits Messdaten bzw. gespeicherte Werte zur Anzeige zu bringen und andererseits eine Eingabe durch einen Benutzer zu ermöglichen. Beispielsweise kann das Steuerelement das Umschalten zwischen dem ersten Speichermodus und dem zweiten Speichermodus ermöglichen.

Insbesondere sind für die Durchführung erfindungsgemässer Verfahren Geräte zur kontinuierlichen Glukoseüberwachung ("Continuous Glucose Monitoring" - CGM) besonders geeignet. CGM bezeichnet eine neue Technologie für die Selbstüberwachung einer Diabeteserkrankung. Derartige Geräte zeichnen sich insbesondere dadurch aus, dass einerseits eine Glukosekonzentration im Körper über Zeiträume von einigen Stunden bis zu einigen Tagen oder gar Wochen resp. Monaten hinweg aufgezeichnet wird. Andererseits ist die Häufigkeit einer Messung viel grösser (Minutenbereich) als bei herkömmlichen Blutglukose-Messverfahren (Stunden). Indem diese zeitlich gut aufgelösten Daten gespeichert werden, kann der Patient und/oder ein Gesundheitsverantwortlicher des Patienten die Daten für eine Anpassung einer Therapie heranziehen und gegebenenfalls beispielsweise eine Anpassung von Essgewohnheiten bzw. einer Medikamentierung etc. des Patienten in die Wege leiten. Ausserdem kann durch die Speicherung ein persönliches Datenarchiv erstellt werden, welches beispielsweise eine Langzeit-Analyse patientenindividueller metabolischer Reaktionen auf gewisse Ereignisse als zeitlich hochaufgelöste Sequenzen bzw. Formen oder Muster erlaubt.

Die Vorrichtung umfasst zur Messung eines Glukosespiegels bevorzugt einen kontinuierlich arbeitenden Glukosesensor, welcher im oder am menschlichen Körper angeordnet ist, um eine Glukosekonzentration in Körperflüssigkeiten zu messen. Die Messwerte werden in der Vorrichtung abgespeichert oder können an andere Komponenten zur Datenverarbeitung oder -visualisierung übermittelt werden, insbesondere z. B. an PDAs, PCs, Mobiltelefone oder Fernbedienungen für Insulinpumpen bzw. direkt an Insulinpumpen. Der Sensor kann z. B. auch in Analysegeräte wie beispielsweise tragbare CGM-Geräte integriert bzw. an derartige Geräte angeschlossen sein. Bevorzugt umfassen derartige Analysegeräte auch eine Anzeige, insbesondere einen Bildschirm, auf welchem aktuelle sowie gespeicherte Daten zur Ansicht gebracht werden können. Insbesondere sind derartige Geräte bevorzugt derart ausgebildet bzw. mit sämtlichen Komponenten versehen, welche für die Durchführung erfindungsgemässer Verfahren erforderlich sind.

Um die von der Messvorrichtung kontinuierlich gemessenen Werte für einen intermediären Zugriff zwischenspeichern zu können umfasst die Vorrichtung bevorzugt einen Pufferspeicher. Der Pufferspeicher ist dabei derart ausgebildet, dass andere Komponenten der Vorrichtung auf ihn zugreifen können, um bei Bedarf zwischengespeicherte Messwerte entnehmen oder Einspeisen zu können. Der Pufferspeicher kann direkt in die Systemsteuerung integriert sein, ist aber mit Vorteil direkt in die Messvorrichtung integriert. Insbesondere ist ein derartiger Zwischen- bzw. Pufferspeicher derart ausgebildet, dass eine Mehrzahl von Messwerten speicherbar ist, welche vor einem aktuellen Zeitpunkt ermittelt worden sind. Die Mehrzahl von Messwerten im Pufferspeicher entspricht somit einem gemessenen Verlauf der Messkurve in einem Zeitfenster vor dem aktuellen Zeitpunkt. Hierfür geeignete Zwischenspeicher arbeiten bevorzugt nach einem First-In-First-Out (FIFO) Prinzip bzw. nach einem Eimerketten-Prinzip. Bei derartigen Speicherarchitekturenwerden diejenigen Inhalte des Pufferspeichers, die zuerst gespeichert wurden, auch zuerst wieder aus dem Pufferspeicher entnommen bzw. gelöscht oder überschrieben.

Weiter umfasst eine erfindungsgemässe Vorrichtung mit Vorteil eine Auswertungsvorrichtung, welche eine Analyse, insbesondere einer Variabiliät, der gemessenen Werte erlaubt. Die Auswertungsvorrichtung entnimmt beispielsweise zwischengespeicherte Messwerte aus dem Puffer und bestimmt aufgrund einer Analyse der entnommenen Messwerte und allenfalls aktueller Messwerte eine aktuelle Variabilität der Messkurve. Aufgrund der Analyse kann dann durch die Systemsteuerung beispielsweise ein weiterer Verlauf der Messkurve extrapoliert bzw. geschätzt und/oder ein Zeitraum bis zur nächsten Speicherung eines Messwerts angepasst werden.

Die Systemsteuerung umfasst weiter mit Vorteil einen Rechner zur Berechnung von Trendwerten über den zu erwartenden zukünftigen Verlauf des gemessenen Parameters. In einer bevorzugten Ausführungsform ist der Rechner zudem derart ausgebildet und programmiert, dass eine zeitliche Ableitung der Messwerte berechnet werden kann. Mit Vorteil ist der Rechner derart ausgebildet und programmiert, dass auch eine zweite zeitliche Ableitung der Messwerte, d. h. eine erste zeitliche Ableitung der Trendwerte, errechnet werden kann. Der Rechner wirkt dabei insbesondere derart mit den weiteren Komponenten der Vorrichtung zusammen, dass die Ableitungen der Messwerte bei Bedarf abgespeichert werden können.

Zur Ausführung eines der oben genannten Verfahren kann ein Computerprogrammprodukt zur Anwendung kommen, welches Daten einer kontinuierlich an oder in einem menschlichen Körper durchgeführten Messung eines Parameters verarbeitet, wobei der gemessene Parameter insbesondere eine Glucosekonzentration beschreiben kann. Insbesondere ist das Computerprogramm für eine Durchführung auf einer Datenverarbeitungsvorrichtung geeignet. Dies kann insbesondere ein handelsüblicher Laptop sein, an welchen eine oben beschriebene Messvorrichtung angeschlossen ist oder das Computerprogramm kann auf einem speziell dafür vorgesehenen tragbaren Gerät ausgeführt werden.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen schematisch:
- Fig. 1: Ein Blockdiagramm eines erfindungsgemässen Verfahrens zur Speiche- rung von Messwerten;
- Fig. 2: einen Verlauf einer Glukosekonzentration und ein Diagramm zum erfin- dungsgemässen Speicherverfahren;
- Fig. 3: einen konkreten Verlauf einer Glukosekonzentration in einem Menschen im Laufe von 24 Stunden;
- Fig.4: eine erste zeitliche Ableitung des Glukosekonzentrationsverlaufs der Fig. 3;
- Fig. 5: eine zweite zeitliche Ableitung des Glukosekonzentrationsverlaufs der Fig. 3;
- Fig. 6: einen Verlauf einer Glukosekonzentration über 4 Stunden;
- Fig. 7: eine Vorrichtung zur Durchführung eines erfindungsgemässen Verfah- rens.

### Grundsätzlich sind in den Figuren gleiche Teile mit gleichen Bezugszeichen versehen.

### Wege zur Ausführung der Erfindung

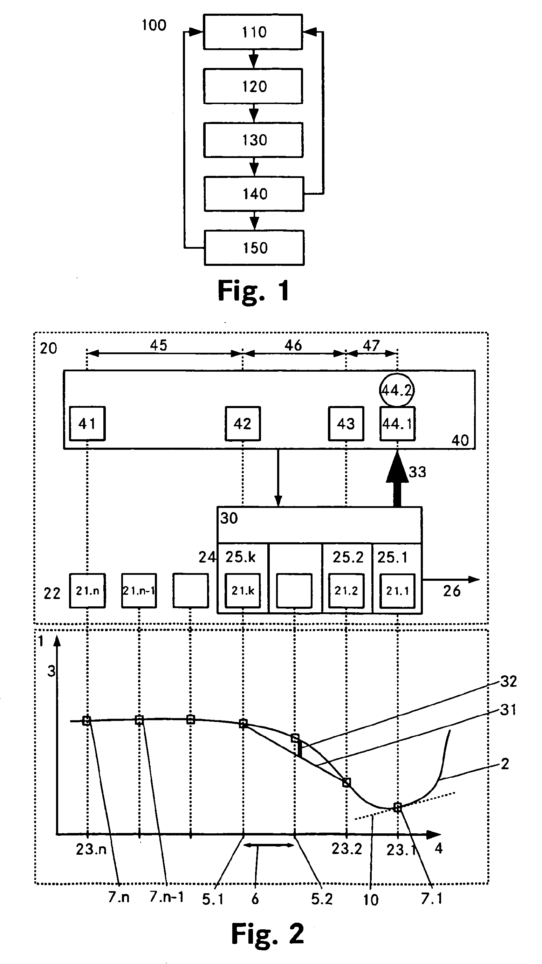

Figur 1 zeigt ein Blockdiagramm 100 einer beispielhaften Variante des erfindungsgemässen Verfahrens zur Speicherung von Messwerten. Das Verfahren umfasst in einem ersten Schritt 110 die Messung eines physiologischen Parameters, insbesondere an oder in einem menschlichen Körper. Gemäss dem Verfahren wird der so gemessene Wert in einem weiteren Schritt 120 des Verfahrens in einem Pufferspeicher zwischengespeichert. Der Pufferspeicher umfasst dabei vorzugsweise eine Anzahl von Registern, in welchen eine Reihe von Messwerten verlustfrei zwischengespeichert und zur Verfügung gestellt werden können. In einem nächsten Schritt 130 des Verfahrens erfolgt eine Analyse der zwischengespeicherten Messwerte wobei auch bereits gespeicherte Werte aus einem Speicher beigezogen werden können. Mit der Analyse 130 wird insbesondere eine Variabilitätsanalyse der gepufferten Messwerte vorgenommen und/oder eine Trendanalyse bzw. eine Analyse von Trendänderungen. In einem weiteren Schritt 140 wird eine Relevanz eines gepufferten Messwertes, beispielsweise eines zuletzt gemessenen Wertes, für den Verlauf der Messkurve geprüft, indem ein Speicherkriterium erzeugt und einem gepufferten Messwert zugeordnet wird. Das Speicherkriterium wird geprüft

Bei Erfüllung des Speicherkriteriums wird der dem Speicherkriterium zugeordnete Messwert des Puffers in einem weiteren Schritt 150 des Verfahrens in einem Speicher abgelegt. Es ist dabei auch denkbar, dass ein Speicherkriterium ein Ensemble von mehreren Messwerten betrifft, welche sämtlich abgespeichert werden, wenn das Kriterium erfüllt ist. Danach beginnt das Verfahren mit der Ermittlung eines weiteren Messwerts wieder bei Schritt 110.

Ist kein Speicherkriterium erfüllt, so beginnt das Verfahren ohne Durchführung des Speicherschritts 150 wieder mit Schritt 110. Die Messperiode, mit welcher sich das Verfahren wiederholt ist dabei derart gewählt, dass der Verlauf des physiologischen Parameters weitgehend aufgelöst wird.

Figur 2 zeigt einen Ausschnitt eines schematischen Diagramms 1 eines gemessenen Verlaufs 2 eines physiologischen Parameters, insbesondere eine Glucosekonzentration, beispielsweise in einem menschlichen Körper. Auf einer Ordinate 3 des Diagramms 1 ist dabei die Glucosekonzentration in beliebigen Einheiten aufgetragen, während auf der Abszisse 4 die Zeit von links nach rechts zunehmend aufgetragen ist. Zwei aufeinander folgende Teilstriche 5.1 und 5.2 der Abszisse begrenzen dabei ein Zeitintervall, welches einer Messperiode 6 entspricht, mit welcher kontinuierlich Werte 7.1 bis 7.n des Kurvenverlaufs 2 bestimmt werden. Die Werte 7.1 bis 7.n geben den Verlauf 2 der gemessenen Kurve vergleichsweise genau wieder.

Zusätzlich ist in Fig. 2 ein abstrahiertes Diagramm 20 der Komponenten für eine Verarbeitung, insbesondere eine Speicherung, der Werte 7 des Verlaufs 2 dargestellt. Die dargestellten Komponenten dienen hierbei zur Illustration einer Variante des erfindungsgemässen Verfahrens. Im Diagramm 20 sind durch das Verfahren gemessene Messwerte 21.1 bis 21.n, welche den Messungen der Kurvenwerte 7.1 bis 7.n entsprechen, als abstrakte Informationsblöcke einer Messreihe 22 dargestellt. Die Messwerte 21 sind dabei parallel zur Zeitachse 4 des Diagramms 1 entsprechend ihrem Messzeitpunkt 23.1 bis 23.n in einer Reihe angeordnet.

Bei der Durchführung des Verfahrens werden jeweils eine Anzahl von k Messwerten, im vorliegenden Fall die Messwerte 21.1 bis 21.k, in einem Zwischenpuffer 24 abgelegt, um einen Zugriff auf wenigstens einen Teil bereits ermittelter Messwerte 21.1 bis 21.n zu ermöglichen. Der Zwischenpuffer 24 funktioniert dabei bevorzugt nach einem bekannten Eimerketten- oder FIFO-Prinzip. Hierzu sind eine Anzahl von Registern 25.1 bis 25.k im Zwischenpuffer 24 vorgesehen, wobei bei einer neuen Ermittlung eines neuen Messwertes 21.1 der Inhalt eines letzten Registers 25.k durch den Inhalt des vorletzten Registers 25.k-1 überschrieben wird. Dieser Vorgang wird über alle Register durchgeführt, bis ein Inhalt des Registers 25.2 durch den Inhalt des Registers 25.1 überschrieben ist. In das erste Register 25.1 wird dann der neu gemessene Wert 21.1 geschrieben. Der Zwischenpuffer 24 tastet also die gesamte Messreihe 22 in Form eines Zeit- bzw. Messfensters im Wesentlichen der Länge k Messperioden in Richtung wachsender Zeit 26 ab ("moving window").

Bei der Durchführung des erfindungsgemässen Verfahrens gemäss der Fig. 2 wird in einer Auswertungsvorrichtung 30 bei Bedarf eine Analyse der im Puffer 24 gespeicherten Messwerte 21.1 bis 21.k durchgeführt, wobei auch in einem Speicher 30 abgelegte Werte 41 bis 44 beigezogen werden können. Aufgrund der Analyse findet in der Auswertungsvorrichtung 30 eine Prüfung eines oder mehrerer Speicherkriterien statt, auf deren Basis entschieden wird, ob ein im Puffer 24 vorhandener, im vorliegenden Fall ein zuletzt gemessener, Messwert 21.1 im Speicher 40 abgelegt werden soll. Derartige Speicherkriterien können fest vorgegeben sein (fest vorgegebene Zeitabstände) oder, wie im Falle der Darstellung der Fig. 2, adaptiv auf der Basis des aktuellen und vorherigen Kurvenverlaufs 2 bestimmt werden. Gemäss der Darstellung der Fig. 2 wird in der Auswertungsvorrichtung 30 in einem Auswertungsschritt auf die Register 25.1 bis 25.k des Puffers 24 zugegriffen und es werden die dort gespeicherten Messwerte 21.1 bis 21.k analysiert und mit den im Speicher 40 gespeicherten Werte 41 bis 44 verglichen. Aus den zwischengespeicherten Messwerten 21.1 bis 21.k oder einer Teilmenge davon werden eine erste Ableitung sowie eine zweite zeitliche Ableitung des Kurvenverlaufs 2 bestimmt. Zudem werden eine Kombination der gepufferten Messwerte 21.1 bis 21.k und der gespeicherten Werte 41 bis 44 für eine bereichsweise lineare Interpolation des Kurvenverlaufs 2 herangezogen.

Ein erstes Speicherkriterium gilt dabei als erfüllt, wenn die zweite zeitliche Ableitung zum Zeitpunkt 23.1 einer aktuellen Messung 21.1 einen gewissen vorgegebenen Wert übersteigt. Dies bedeutet, dass die Steigung 10 selbst zwar klein sein kann, aber eine Krümmung der Messkurve 2 einen gewissen Wert übersteigt und demnach vergleichsweise gross ist. In diesem Fall wird der aktuelle Messwert 21.1 zusammen mit der momentanen Steigung 10 der Messkurve 2, d. h. der zeitlichen Ableitung 10 zum Zeitpunkt 23.1 der Ermittlung des Messwerts 21.1, als Wertepaar 44.1/44.2 im Speicher 40 abgelegt.

Ein zweites Speicherkriterium wird aufgrund einer linearen Interpolation 31 erzeugt. In der Fig. 2 ist beispielsweise zu einem früheren Zeitpunkt 23.2 der Messwert 21.2 aufgrund des Interpolationskriteriums als Wert 43 abgespeichert worden. Zum Zeitpunkt 23.2 stellte der Messwert 21.2 den zuletzt gemessenen Messwert dar und der zuletzt gespeicherte Messwert 21.k ist als gespeicherter Wert 42 im Speicher 40 abgelegt. Eine lineare Interpolation zwischen Wert 42 und Messwert 21.2 ergibt die gezeigte Linie 31. Ein Abstand der Interpolation 31 von der Messkurve 2, insbesondere ein Maximum des Abstandes, wird als Fehlermass herangezogen. Im vorliegenden Fall überstieg das Fehlermass bzw. der Abstand 32 einen vorgegebenen Wert, wodurch das Interpolations-Speicherkriterium für den Messwert 21.2 erfüllt war und eine Speicherung als gespeicherter Wert 43 erfolgte.

Wie aus der Fig. 2 ersichtlich wird, ergibt sich somit ein variabler Zeitabstand der gespeicherten Werte 41 bis 44. Ein erster Abstand 45 zwischen den aufeinander folgenden gespeicherten Werten 41 und 42 beträgt das (n-k)-fache der Messperiode 6, während ein Abstand 46 zwischen den gespeicherten Werten 42 und 43 das (k-2)-fache der Messperiode 6 beträgt. Zwischen den beiden letzten gespeicherten Werten 43 und 44 liegt ein Zeitabstand 47 von einer Messperiode 6.

Wie aus der Fig. 2 ersichtlich wird, befinden sich weniger gespeicherte Werte im Speicher 40 als in der gesamten kontinuierlich ermittelten Messreihe 22. Die Messwerte 21.k+1 bis 21.n-1 sowie die Messwerte 21.3 bis 21.k-1 wurden aufgrund vergleichsweise geringer Relevanz für den Kurvenverlauf 2 durch das erfindungsgemässe Verfahren ausgesondert und nicht im Speicher 40 abgelegt.

Während das erläuterte Beispiel der Fig. 2 mehrere Speicherkriterien für einen aktuellen Messwert prüft, können erfindungsgemässe Verfahren auch nur einzelne Speicherkriterien prüfen. Eine Kombination wurde im vorliegenden Fall zur umfassenderen Illustration des erfindungsgemässen Verfahrens gewählt. Zudem ist anzumerken, dass ein Speicherkriterium nicht notwendigerweise nur einem einzelnen wie z. B. einem zuletzt gemessenen Messwert zugeordnet werden kann, sondern beispielsweise auch einem Ensemble von Messwerten, welches aufgrund des Speicherkriteriums abgespeichert werden oder nicht.

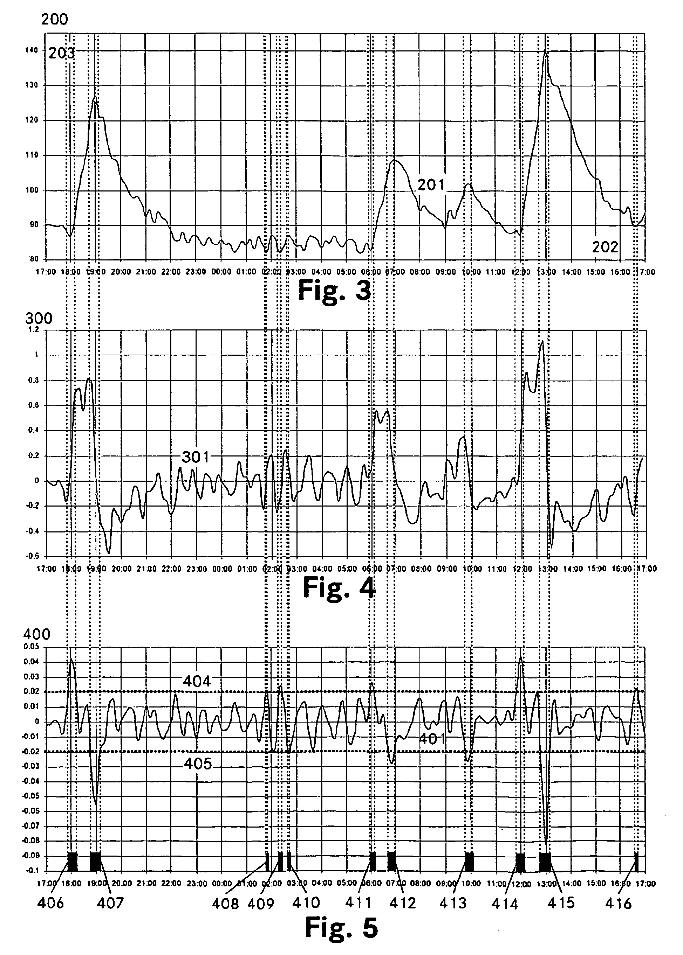

Das Diagramm 200 der Fig. 3 zeigt einen simulierten Verlauf 201 einer Glukosekonzentration eines Menschen im Verlauf von 24 Stunden. Auf der Abszisse 202 ist die Zeit als Uhrzeit von 17:00 Uhr am ersten Tag bis 17:00 Uhr am nächsten Tag aufgetragen. Auf der Ordinate 203 ist die Glukosekonzentration in Einheiten von Milligramm pro Deziliter dargestellt. Der dargestellte Wertebereich umfasst dabei einen Bereich von etwa 80 bis ca. 140 Einheiten.

Um ca. 17:00 Uhr befindet sich die Glukosekonzentration im Bereich von ca. 90 Einheiten. Um etwa 18:00 Uhr ist ein Einbruch der Glukosekonzentration im Blut ersichtlich. Eine um ca. 18:00 Uhr eingenommene Mahlzeit hebt den Glukosespiegel innerhalb etwa einer Stunde im Wesentlichen linear auf einen lokalen Maximalwert von etwa 128 Einheiten an. Bis ca. 22:00 Uhr fällt die Glukosekonzentration weitgehend exponentiell auf einen Minimalwert von im Mittel etwa 85 ab. Um diesen Wert oszilliert die Glukosekonzentration bis zum nächsten Morgen um 6:00 Uhr mit geringer Amplitude (etwa 3 Einheiten). Ein Frühstück um ca. 6:00 Uhr lässt den Glukosespiegel in der Zeit einer Stunde, im Wesentlichen linear, auf einen Wert von ca. 110 Einheiten ansteigen und in der Folge wieder weitgehend exponentiell bis 9:00 Uhr auf einen Wert von ca. 90 Einheiten abfallen. Eine Zwischenmahlzeit hebt den Glukosespiegel, wieder im Wesentlichen linear, im Verlauf einer Stunde auf einen Wert von ca. 102 Einheiten, worauf erneut ein weitgehend exponentieller Abfall auf ca. 88 Einheiten um 12:00 Uhr stattfindet. Ein um etwa 12:00 Uhr eingenommenes Mittagessen lässt den Glukosespiegel innerhalb einer Stunde im Wesentlichen linear auf einen Höchstwert von ca. 141 Einheiten steigen, worauf ein weitgehend exponentieller Abfall auf rund 94 Einheiten am Ende des dargestellten 24 Stunden Profils stattfindet. Um ca. 16:30 Uhr erfolgt zuvor ein kleiner Einbruch des Glukosespiegels 201 auf etwa 90 Einheiten.

Ein Diagramm 300 der Fig. 4 zeigt eine erste zeitliche Ableitung 301 des im Diagramm 200 der Fig. 3 gezeigten Glukosekonzentrationsverlaufs 201 und ein Diagramm 400 der Fig. 5 zeigt eine zweite zeitliche Ableitung 401 der Kurve 201. Im Diagramm 400 der Fig. 5 sind zudem ein positiver Minimalwert 404 und ein negativer betragsmässiger Minimalwert 405 für die zweite Ableitung 401 eingezeichnet. Die Beträge der gewählten Minimalwerte 404 und 405 sind dabei rein illustrativ und können in einer konkreten Umsetzung des Verfahrens deutlich abweichen. Zeitabschnitte 406-416, in welchen die zweite Ableitung 401 die Minimalwerte überschreitet, sind in allen drei Diagrammen 200, 300 und 400 eingezeichnet, in Fig. 5 aber durch geschwärzte Bereiche besonders hervorgehoben.

Ein Vergleich der Lage der Bereiche 406 bis 416 mit dem Kurvenverlauf 201 des Diagramms 200 zeigt deutlich, dass die zweite zeitliche Ableitung des Glukosekonzentrationsverlaufs 201 ein besondere gutes Kriterium ist, um Stellen grosser Änderung im Verlauf 201 zu detektieren. Der Bereich 406 bezeichnet beispielsweise die zeitliche Lage des oben beschriebenen Einbruchs um etwa 18:00 Uhr, während die zeitliche Lage des Bereichs 407 das lokale Maximum um etwa 19:00 bezeichnet. Ebenso finden sich korrespondierende starke Änderungen in der Glukosekonzentration 201 auch für die Bereiche 411 und 412 (etwa 6:00 Uhr und 7:00 Uhr), 413 (ca. 10:00 Uhr), 414 und 415 (ca. 12:00 Uhr und 13:00 Uhr) und bei 416 (etwa 16:30 Uhr). Die Bereiche 408 bis 410 betreffen Änderungen während der oben beschriebenen nächtlichen Oszillationen um einen im Wesentlichen konstanten Mittelwert.

Gemäss einer Variante des erfindungsgemässen Verfahrens werden in den Bereichen 406 bis 416 bevorzugt Wertepaare abgespeichert, welche einerseits den Glukosewert und andererseits die gleichzeitig ermittelte erste zeitliche Ableitung umfassen. Aufgrund derartiger gespeicherter Wertepaare kann eine gute Kenntnis über den sich stark ändernden Verlauf der Glukosekonzentration 201 in den Bereichen 406 bis 416 gewonnen werden. Insbesondere kann damit ein Verlauf 201 aus den gespeicherten Werten mit guter Genauigkeit rekonstruiert werden.

Aufgrund der betragsmässig vergleichsweise kleinen zweiten Ableitung in Zeiträumen ausserhalb der Bereiche 406 bis 416 sind keine wesentlichen Änderungen eines Trends der Kurve 201 zu erwarten. In diesen Bereichen kann die Kurve 201 daher im Nachhinein aufgrund nur weniger gespeicherter Werte mit vergleichsweise guter Genauigkeit rekonstruiert werden. Dabei ist eine Speicherung der Messwerte ohne erste Ableitung bzw. eines Trends in den meisten Fällen hinreichend, da keine wesentliche Änderung der Kurvensteigung bzw. des Trends zu erwarten ist. Zudem weist in Zeiträumen ausserhalb der Bereiche 406-416 der Verlauf 201 in grober Näherung ein weitgehend *a priori* bekanntes Verhalten auf (z. B. linearer Anstieg, exponentieller Abfall), welches ebenfalls in das Verfahren einbezogen werden kann.

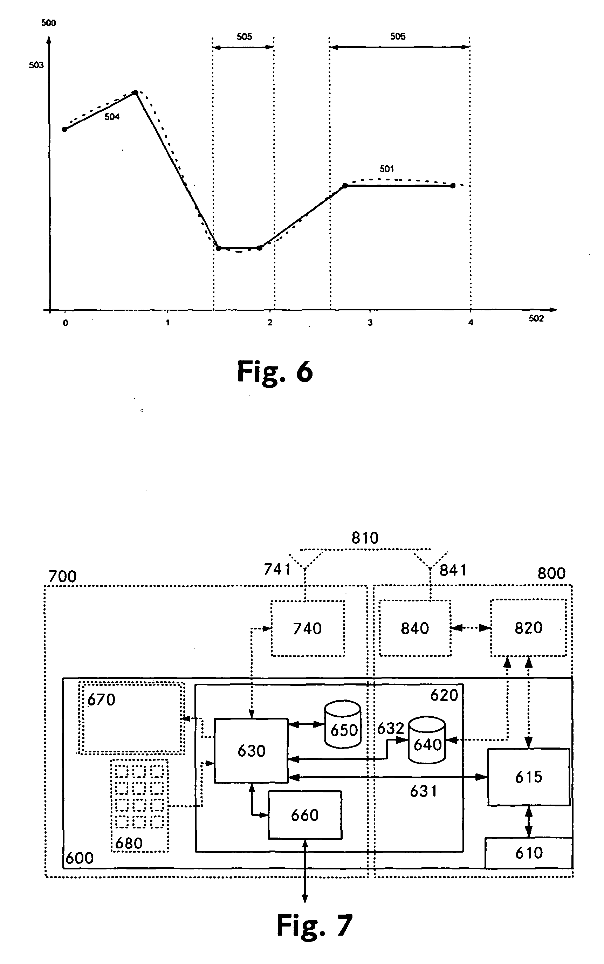

Figur 6 zeigt einen schematischen Verlauf 501 einer gemessenen Glukosekonzentration über 4 Stunden hinweg. Ein dargestelltes Diagramm 500 weist eine Abszisse 502 und eine Ordinate 503 auf, wobei auf der Abszisse 502 die Zeit ansteigend aufgetragen ist und auf der Ordinate 503 die Glukosekonzentration. Der gemessene Verlauf 501 ist dabei gestrichelt dargestellt, während ein rekonstruierter Verlauf 504 durch 6 gespeicherte Werte, welche linear interpoliert sind, angedeutet ist. Die folgende Tabelle 1 gibt die durch das erfindungsgemässe Verfahren erreichte Reduktion der Speicherrate gegenüber einer vollständigen Speicherung der gesamten Messreihe über 4 Stunden an (letzte Zeile, 1-Minuten-Intervall). Der Verlauf 501 weist dabei Zeitabschnitte 505 und 506 geringer Dynamik auf und eine hohe Dynamik sonst. Die zweite Spalte der Tabelle 1 gibt dabei den Speichermodus an: Modus 1 für Speicherintervalle variabler Länge (z. B. adaptiv an den Verlauf der Messkurve angepasst oder aufgrund *a priori* Wissens über die Messkurve festgelegt) und Modus 2 für Speicherintervalle konstanter Länge. Während die Verfahren der Beispiele A-B und D-F nur einen einzigen der beiden Speichermodi anwenden, finden im Beispiel C beide Modi Verwendung. Hierbei wird insbesondere *a priori* Wissen über den zukünftigen Verlauf der Glukosekonzentration für die Festlegung von Zeitbereichen genutzt, in welchen entweder Speichermodus 1 oder Speichermodus 2 zur Anwendung kommt. In Bereichen voraussichtlicher hoher Dynamik (z. B. Einnahme von Mahlzeiten) ist ein fixer Messintervall von 5 Minuten vorgegeben (Speichermodus 2), um ein gutes Sampling des Konzentrationsverlaufs zu gewährleisten. In Bereichen voraussichtlicher geringer oder fehlender Dynamik (z. B. nachts) reicht es, nur sporadisch Messwerte in variablen Zeitabständen zu ermitteln, in Beispiel C ein Messwert pro dynamikloses Zeitintervall, da sich der Glukosespiegel in diesen Zeiträumen nicht wesentlich ändert.

In anderen Varianten des Verfahrens kann beispielsweise zwischen Speichermodi aufgrund einer Variabilitätsanalyse der Messkurve umgeschaltet werden. Beispielsweise benutzt das Verfahren, sobald eine hohe Dynamik festgestellt wird, den Speichermodus 2 und bei Detektierung einer geringen Dynamik kommt Speichermodus 1 zur Anwendung. Es kann aber auch eine Anwendung des Speichermodus 1 in Bereichen einer hohen Dynamik vorteilhaft sein und ebenso eine Anwendung des Speichermodus 2 in Bereichen einer vergleichsweise geringen bis fehlenden Dynamik.

**Tabelle 1: Speichermenqenreduktion durch das Speicherverfahren**

| **Nr.** | **Modus** | **Speicherkriterium** | **Gespeicherte Werte** | **Reduktion** |
|---|---|---|---|---|
| A | 1 | lin. Interpolation | 6 Messwerte | 95% |
| | | Trendinformation | 6 Zeitwerte | |
| B | 1 | lin. Interpolation | 6 Messwerte | 92.5% |
| | | Trendinformation | 6 Trendwerte | |
| | | | 6 Zeitwerte | |
| C | 1+2 | lin. Interpolation | 24 Messwerte | 88 % |
| | | *a priori* Wissen über Verlauf | (5 Min. Intervall bei Dynamik) | |
| | | | 2 Messwerte | |
| | | | 2 Zeitwerte (ohne Dynamik) | |
| D | 2 | 15 Min. Intervall | 16 Messwerte 16 Trendwerte | 87 % |
| E | 2 | 5 Min. Intervall | 48 Messwerte | 80 % |
| F | 2 | 1 Min. Intervall | 240 Messwerte | 0 % |

Figur 7 zeigt eine schematische Ansicht eines erfindungsgemässes Geräts 600 zur Verarbeitung bzw. Speicherung einer Reihe von Messwerten eines zeitabhängigen Parameters, welcher in oder an einem menschlichen Körper gemessen wird, insbesondere eine Glukosekonzentration in einer Körperflüssigkeit wie Blut. Die Anordnung umfasst dabei einen Glukosesensor 610 sowie eine Verarbeitungseinheit bzw. eine Systemsteuerung 620. Im dargestellten Beispiel misst der Glukosesensor 610 beispielsweise als enzymatischer Sensor elektrochemisch (oder auch photometrisch) eine Glukosekonzentration in einer Körperflüssigkeit. Es können als Glukosesensor 610 aber auch Affinitätssensoren zur Anwendung kommen, die optisch (also auch photometrisch) oder aber fluidisch (Viskositätsmessung oder osmotisch, bzw. andere fluidische Parameter) ausgewertet werden.

Gemäss dem dargestellten Beispiel umfasst die Systemsteuerung 620 eine zentrale Verarbeitungseinheit 630 (CPU- Central Processing Unit) sowie einen Zwischenspeicher 640 und einen Speicher 650. Eine Schnittstelle 660 der Systemsteuerung 620 ermöglicht beispielsweise einen Anschluss des Geräts 600 an externe Peripherieeinrichtungen wie Datenverarbeitungsanlagen, z. B. Personal Computer (PC) eines Patienten oder eines Gesundheitsverantwortlichen des Patienten, oder aber auch den Anschluss an beispielsweise eine externe Insulinabgabevorrichtung, Personal-Digital-Assistants (PDAs) oder Mobiltelefone zur Datenfernübertragung.

Der Messsensor 610 ist im dargestellten Beispiel mir einer Analogelektronik 615 verbunden, welche das Sensorsignal des Messsensors 610 wandelt und der zentralen Verarbeitungseinheit 630 zur Verfügung stellt. Der CPU 630 ist über eine Leitung 631 mit der Analogelektronik 615 sowie über eine Leitung 632 mit dem Zwischenspeicher 640 verbunden. Zusätzlich weist der CPU 630 Verbindungen mit dem Speicher 650 und der Schnittstelle 660 auf. Der CPU 630 steuert dabei den Sensor 610 und speichert periodisch mit einer Messperiode einen gemessenen Glukosewert im Zwischenspeicher 640. Bei der Durchführung des erfindungsgemässen Verfahrens entnimmt der CPU 630 Messwerte aus dem Zwischenspeicher 640, bestimmt Speicherkriterien und legt Messwerte, für welche ein Speicherkriterium erfüllt ist, im Speicher 650 ab.

Hierbei können zusätzlich Anzeigemittel, z. B. in Form eines grafischen Displays 670, sowie Steuerelemente, beispielsweise in Form einer Tastatur 680, vorgesehen sein, wobei das Display 670 eine grafische Darstellung von im Speicher 650 abgelegten Werten ermöglicht und die Tastatur 680 eine Interaktion mit dem Gerät 600 erlaubt. Beispielsweise kann über die Tastatur 680 ein Anzeigemodus des Displays 670 gewechselt werden oder aber auch in das auf dem CPU 630 ausgeführte erfindungsgemässe Verfahren eingegriffen werden, indem z. B. ein Speichermodus gewechselt wird.

In einer Abwandlung der dargestellten Ausführungsform des Geräts 600 umfasst eine erfindungsgemässe Vorrichtung eine Steuereinheit und eine damit verbundene Messeinheit, welche jeweils als eingeständiges Gerät 700 (Steuereinheit) bzw. 800 (Messeinheit) ausgeführt sind. Die Messeinheit 800 ist dabei über eine drahtlose RF-Verbindung 810 an die Steuereinheit 700 angebunden. Es versteht sich, dass eine drahtlose Verbindung beispielsweise auch über optische, akustische, elektrostatische (kapazitive) und/oder induktive Verfahren hergestellt werden kann. In anderen Ausführungsformen können die Messeinheit und die Steuereinheit auch drahtgebunden miteinander verbunden sein. Die beschriebene drahtlose RF-Verbindung 810 stellt somit nur eine spezielle Variante möglicher Ausführungen mit getrennten Mess- und Steuereinheiten dar.

Die Steuereinheit 700 umfasst den CPU 630, den Speicher 650 sowie die Schnittstelle 660, wobei wie beim Gerät 600 optional das Display 670 und/oder die Tastatur 680 vorhanden sein können. Diese Komponenten sind gemäss der integrierten Ausführung des Geräts 600 miteinander verbunden. Zudem weist die Steuereinheit 700 eine Schnittstelle 740 mit einer Antenne 741 zum Empfang bzw. auch zum Senden von RF-Signalen auf.

Die Messeinheit 800 umfasst den Zwischenspeicher 640 zur Zwischenspeicherung von Messwerten, welche mit dem ebenfalls von der Messeinheit 800 umfassten Sensor 610 werden. Bevorzugt weist die Messeinheit 800 eine eigene zentrale Recheneinheit (CPU) 820 auf, welche mit dem Zwischenspeicher 640 sowie mit der ebenfalls in der Messeinheit 800 vorgesehenen Analogelektronik 615 verbunden ist. Die Analogelektronik 615 ist dabei mit dem Glukosesensor 610 verbunden und stellt dessen gewandelte Sensorsignale dem CPU 820 zur Verfügung. Weiter umfasst die Messeinheit 800 zur Datenübertragung eine Schnittstelle 840 mit einer Antenne 841. Der CPU 820 steuert den Sensor 610 derart, dass periodisch Messwerte im Zwischenspeicher 640 zwischengespeichert werden.

Im Gegensatz zum integrierten Gerät 600 weist der CPU 630 in der dargestellten Ausführungsform der Vorrichtung mit getrennter Steuereinheit 700 und Messeinheit keine direkte Verbindung mit dem Zwischenspeicher 640 und dem Sensor 610 der Messeinheit 800 auf. Daten der Messeinheit 800 sowie deren Komponente werden ausschliesslich über die drahtlose RF-Verbindung 810 an die Steuereinheit 700 übertragen bzw. gesendet. Hierbei kann die drahtlose RF-Verbindung 810 je nach Anforderung bidirektional ausgeführt sein oder aber auch nur eine Datenübertragung von der Messeinheit 800 an die Steuereinheit 700 erlauben.

Die im Zwischenspeicher 640 abgelegten Messwerte werden vorzugsweise in periodischen Abständen an die Steuereinheit 700 übermittelt. Hierzu werden die Messwerte im Zwischenspeicher 640 durch den CPU 820 ausgelesen und an die Schnittstelle 840 übermittelt, dort beispielsweise verschlüsselt, und von der Schnittstelle 840 mit der Antenne 841 über die drahtlose Verbindung 810 an die Steuereinheit 700 übermittelt.

Das RF-Signal 810 der Messeinheit 800 wird über die Antenne 741 der Steuereinheit 700 empfangen und von der Schnittstelle 740 weiterverarbeitet, beispielsweise entschlüsselt. Die empfangenen Messwerte werden dann von der Schnittstelle 740 an den CPU 630 übermittelt, welcher gemäss dem erfindungsgemässen Verfahren ausgewählte Messwerte im Speicher 650 ablegt. Auf entsprechender Anfrage, beispielsweise durch eine externe Datenverarbeitungsanlage oder aufgrund einer Eingabe durch einen Benutzer, kann der CPU 630 die gespeicherten Werte aus dem Speicher 650 entnehmen und beispielsweise an die Schnittstelle 660 weitergeben und/oder auf dem Display 670 zur Anzeige bringen.

Das integrierte Gerät 600 hat den Vorteil, dass es nur einen CPU 630 aufzuweisen braucht und eine Verbindung des CPUs 630 mit dem Sensor 610 und dem Zwischenspeicher 640 direkt über Leitungen 670 und 680 erfolgen kann. Dabei müssen aber sämtliche erforderlichen Komponenten in einem einzelnen Gerät untergebracht sein, welches vergleichsweise unhandlich sein kann. Im Gegensatz dazu erlaubt eine Ausführung mit getrennten Einheiten 700 und 800 eine vergleichsweise kleine und unauffällige Gestaltung der Messeinheit 800, welche insbesondere unabhängig von der Steuereinheit 800 am Körper angebracht werden kann. Die Steuereinheit 700 kann dabei beispielsweise unauffällig in einer Mantel- bzw. Hosentasche mitgetragen werden. Dafür sind aber zusätzliche Komponenten wie Schnittstellen 740 und 840 zur Kommunikation und ein zusätzlicher CPU 820 der Messeinheit 800 erforderlich.

Zusammenfassend ist festzustellen, dass das erfindungemässe Verfahren eine selektive Speicherung mit vergleichsweise grosser Genauigkeit in Bereichen unerwarteter Änderungen, d. h. in Bereichen grosser Trendänderungen ermöglicht, während in Bereichen mit geringer Trendänderung und damit einfach vorhersagbarem Verlauf nur vergleichsweise wenige Werte gespeichert werden. Aus der Gesamtheit der nach dem erfindungsgemässen Verfahren gespeicherten Werte, welche eine deutlich geringere Datenmenge ergeben können, als eine komplette Messreihe, kann dennoch der ursprüngliche Verlauf der Messkurve mit guter Genauigkeit rekonstruiert werden. Zudem lassen sich durch die selektive Messwertspeicherung Rauschen und andere unerwünschte Fluktuationen in den gemessenen Werten einfach ausfiltern. Zudem kann zur weiteren Datenreduktion in das Verfahren einerseits problemlos *a priori* Wissen um den zu erwartenden Verlauf der Messkurve einfliessen und andererseits ist das Verfahren zudem derart adaptiv, dass es einen Speichermodus bzw. Speicherintervalle zwischen zu speichernden Werten an den momentanen Verlauf der Messkurve anpassen kann.

## Patentansprüche

1. Verfahren zum Speichern einer Reihe von gemessenen Werten, die einen zeitabhängigen in oder am menschlichen Körper gemessenen Parameter repräsentieren, insbesondere eine physiologische Glukosekonzentration, wobei die Reihe von gemessenen Werten eine erste Anzahl von Werten umfasst und wobei eine zweite Anzahl von gemessenen Werten gespeichert wird, wobei die zweite Anzahl geringer ist als die erste Anzahl, **dadurch gekennzeichnet, dass** ein erster Speichermodus vorgesehen ist, in welchem ein zeitlicher Abstand zwischen aufeinander folgenden gespeicherten Werten variabel ist.

2. Verfahren nach Anspruch 1, **dadurch gekennzeichnet, dass** im ersten Speichermodus der zeitliche Abstand in Abhängigkeit einer zeitlichen Variabilität der gemessenen Werte angepasst wird.

3. Verfahren nach Anspruch 2, **dadurch gekennzeichnet, dass** ein Fehlermass einer Interpolation zwischen einem letzten gespeicherten Wert und einem zuletzt gemessenen Wert in Bezug auf einen entsprechenden Bereich der Reihe von gemessenen Werten gebildet wird, wobei ein Wert des entsprechenden Bereichs der Reihe gespeichert wird, wenn das Fehlermass einen gewissen Maximalwert überschreitet.

4. Verfahren nach Anspruch 3, **dadurch gekennzeichnet, dass** eine lineare Interpolation zwischen dem letzten gespeicherten Wert und dem zuletzt gemessenen Wert vorgenommen wird.

5. Verfahren nach einem der Ansprüche 1 bis 4, **dadurch gekennzeichnet, dass** ein zweiter Speichermodus vorgesehen ist, in welchem der zeitliche Abstand zwischen aufeinander folgenden gespeicherten Werten konstant ist, wobei insbesondere der erste Speichermodus zur Anwendung kommt, wenn eine Variabilität der gemessenen Werte einen vorgegebenen Wert unterschreitet und der zweite Speichermodus zur Anwendung kommt, wenn die Variabilität den vorgegebenen Wert überschreitet.

6. Verfahren nach einem der Ansprüche 1 bis 5, **dadurch gekennzeichnet, dass** aus der Reihe von gemessenen Werten Trendwerte ermittelt werden.

7. Verfahren nach Anspruch 6, **dadurch gekennzeichnet, dass** zur Ermittlung der Trendwerte ein Kalmán-Filter eingesetzt wird.

8. Verfahren nach Anspruch 6 oder 7 und nach einem der Ansprüche 1 bis 5, **dadurch gekennzeichnet, dass** der aktuelle gemessene Wert gespeichert wird, wenn eine dem aktuell gemessenen Wert zugeordnete Änderung des Trendwerts einen vorgegebenen Minimalwert überschreitet.

9. Verfahren nach einem der Ansprüche 6 bis 8, **dadurch gekennzeichnet, dass** neben den gespeicherten Werten auch Trendwerte gespeichert werden.

10. Verfahren nach Anspruch 9, **dadurch gekennzeichnet, dass** aus der Reihe von gemessenen Werten Trendänderungswerte ermittelt werden, die einer zweiten Ableitung der gemessenen Werte entsprechen.

11. Verfahren nach Anspruch 9 oder 10, **dadurch gekennzeichnet, dass** zeitliche Stützstellen vorgesehen werden, an welchen der entsprechende Trendwert und/oder Trendänderungswert, nicht aber der gemessene Wert selbst abgespeichert wird.

12. Vorrichtung zur Durchführung eines Verfahrens nach einem der Ansprüche 1 bis 11, insbesondere eine tragbare Vorrichtung, die Folgendes umfasst:
a) eine Messvorrichtung zum kontinuierlichen Messen und Einspeisen einer ersten Anzahl von gemessenen Werten, die einen zeitabhängigen in oder am menschlichen Körper gemessenen Parameter repräsentieren , insbesondere einer physiologischen Glucosekonzentration,
b) eine Systemsteuerung mit einer Speichereinheit zum Speichern einer zweiten
Anzahl von gemessenen Werten;
wobei die Systemsteuerung derart ausgebildet und programmiert ist, dass die zweite Anzahl kleiner ist als die erste Anzahl; **dadurch gekennzeichnet, dass** c) ein erster Speichermodus vorgesehen ist, in welchem ein zeitlicher Abstand zwischen aufeinander folgenden gespeicherten Werten variabel ist.

13. Vorrichtung nach Anspruch 12, **wobei** ein Pufferspeicher vorgesehen ist, in welchem kontinuierlich gemessene Werte zwischenspeicherbar sind und weiter eine Auswertungsvorrichtung zur Analyse einer Variabilität der gemessenen Werte vorhanden ist.

14. Vorrichtung nach einem der Ansprüche 12 bis 13, **wobei** die Systemsteuerung einen Rechner zur Berechnung von Trendwerten über den zu erwartenden zukünftigen Verlauf des gemessenen Parameters umfasst, insbesondere zur Berechnung einer zeitlichen Ableitung der Messwerte, und bevorzugt zur Bestimmung einer zeitlichen Ableitung der Trendwerte ausgebildet ist.

15. Computerprogrammprodukt, insbesondere zur Durchführung auf einer Vorrichtung gemäss einem der Ansprüche 12 bis 14, **dadurch gekennzeichnet, dass** es bei einer Ausführung auf einer Datenverarbeitungsmaschine das Verfahren nach einem der Ansprüche 1 bis 12 zum Ablauf bringt.

## Claims

1. Method for storing a series of measured values which represent a time-dependent parameter measured in or on the human body, particularly a physiological glucose concentration, wherein the series of measured values comprises a first number of values and wherein a second number of measured values is stored, the second number being smaller than the first number, **characterized in that** a first storage mode is provided, in which an interval of time between successive stored values is variable.

2. Method according to Claim 1, **characterized in that** in the first storage mode the interval of time is adjusted on the basis of a time-based variability for the measured values.

3. Method according to Claim 2, **characterized in that** a measure of error in an interpolation between a last stored value and a most recently measured value is formed for a relevant range of the series of measured values, wherein a value from the relevant range of the series is stored if the measure of error exceeds a certain maximum value.

4. Method according to Claim 3, **characterized in that** linear interpolation is performed between the last stored value and the most recently measured value.

5. Method according to one of Claims 1 to 4, **characterized in that** a second storage mode is provided, in which the interval of time between successive stored values is constant, wherein particularly the first storage mode is used if a variability for the measured values is below a prescribed value, and the second storage mode is used if the variability exceeds the prescribed value.

6. Method according to one of Claims 1 to 5, **characterized in that** trend values are ascertained from the series of measured values.

7. Method according to Claim 6, **characterized in that** a Kalman filter is used to ascertain the trend values.

8. Method according to Claim 6 or 7 and according to one of Claims 1 to 5, **characterized in that** the current measured value is stored if a trend value change associated with the currently measured value exceeds a prescribed minimum value.

9. Method according to one of Claims 6 to 8, **characterized in that** trend values are also stored besides the stored values.

10. Method according to Claim 9, **characterized in that** trend change values corresponding to a second derivation for the measured values are ascertained from the series of measured values.

11. Method according to Claim 9 or 10, **characterized in that** time-based interpolation points are provided, at which the relevant trend value and/or trend change value, but not the measured value itself, is stored.

12. Apparatus for carrying out a method according to one of Claims 1 to 11, particularly a portable apparatus, which comprises the following:
a) a measuring apparatus for continuously measuring and feeding a first number of measured values which represent a time-dependent parameter measured in or on the human body, particularly for a glucose concentration,
b) a system controller having a memory unit for storing a second number of measured values; wherein the system controller is in a form and programmed such that
the second number is smaller than the first number; **characterized in that**
c) a first storage mode is provided, in which an interval of time between successive stored values is variable.

13. Apparatus according to Claim 12, wherein a buffer store is provided which can be used to buffer-store continuously measured values, and also an evaluation apparatus for analyzing a variability for the measured values is present.

14. Apparatus according to one of Claims 12 and 13, wherein the system controller comprises a computer for calculating trend values using the expected future profile of the measured parameter, particularly for calculating a time-based derivation for the measured values, and is preferably designed to determine a time-based derivation for the trend values.

15. Computer program product, particularly for implementation on an apparatus according to one of Claims 12 to 14, **characterized in that** when executed on a data processing machine it carries out the method according to one of Claims 1 to 12.

## Revendications

1. Procédé d'enregistrement d'une série de valeurs mesurées qui représentent un paramètre mesuré en fonction du temps dans ou sur un corps humain, notamment une concentration physiologique de glucose, la série de valeurs mesurées comprenant un premier nombre de valeurs et un deuxième nombre de valeurs mesurées étant enregistré, le deuxième nombre étant plus petit que le premier nombre, **caractérisé en ce qu'**il est prévu un premier mode de mémorisation dans lequel un intervalle de temps entre les valeurs enregistrées successives est variable.

2. Procédé selon la revendication 1, **caractérisé en ce que** dans le premier mode de mémorisation, l'intervalle de temps est adapté en fonction d'une variabilité dans le temps des valeurs mesurées.

3. Procédé selon la revendication 2, **caractérisé en ce qu'**un indice d'erreur d'une interpolation entre une dernière valeur enregistrée et une dernière valeur mesurée en référence à une plage correspondante de la série de valeurs mesurées est formé, une valeur de la plage correspondante de la série étant enregistrée lorsque l'indice d'erreur est supérieur à une valeur maximale donnée.

4. Procédé selon la revendication 3, **caractérisé en ce qu'**une interpolation linéaire est effectuée entre la dernière valeur enregistrée et la dernière valeur mesurée.

5. Procédé selon l'une des revendications 1 à 4, **caractérisé en ce qu'**il est prévu un deuxième mode de mémorisation dans lequel l'intervalle de temps entre des valeurs enregistrées successives est constant, le premier mode de mémorisation étant notamment utilisé lorsqu'une variabilité des valeurs mesurées est inférieure à une valeur prédéfinie et le deuxième mode de mémorisation étant utilisé lorsque la variabilité dépasse la valeur prédéfinie.

6. Procédé selon l'une des revendications 1 à 5, **caractérisé en ce que** des valeurs de tendance sont déterminées à partir de la série de valeurs mesurées.

7. Procédé selon la revendication 6, **caractérisé en ce qu'**un filtre de Kalman est utilisé pour déterminer les valeurs de tendance.

8. Procédé selon la revendication 6 ou 7 et selon l'une des revendications 1 à 5, **caractérisé en ce que** la valeur mesurée actuelle est enregistrée lorsqu'une modification associée à la valeur mesurée actuelle de la valeur de tendance dépasse une valeur minimale prédéfinie.

9. Procédé selon l'une des revendications 6 à 8, **caractérisé en ce que** les valeurs de tendance sont également enregistrées en plus des valeurs enregistrées.

10. Procédé selon la revendication 9, **caractérisé en ce que** des valeurs de modification de la tendance sont déterminées à partir de la série de valeurs mesurées, lesquelles correspondent à une deuxième dérivation des valeurs mesurées.

11. Procédé selon la revendication 9 ou 10, **caractérisé en ce que** des points de référence dans le temps sont prévus, auxquels la valeur de tendance correspondante et/ou la valeur de modification de la tendance, mais non la valeur mesurée elle-même sont enregistrées.

12. Dispositif pour mettre en oeuvre un procédé selon l'une des revendications 1 à 11, notamment un dispositif portable, comprenant :
a) un dispositif de mesure pour la mesure continue et l'injection d'un premier nombre de valeurs mesurées, lesquelles représentent un paramètre mesuré en fonction du temps dans ou sur un corps humain, notamment une concentration physiologique de glucose,
b) une commande de système munie d'une unité de mémoire pour enregistrer un deuxième nombre de valeurs mesurées,
la commande de système étant configurée et programmée de telle sorte que le deuxième nombre est inférieur au premier nombre, **caractérisé en ce que**
c) il est prévu un premier mode de mémorisation dans lequel un intervalle de temps entre les valeurs enregistrées successives est variable.

13. Procédé selon la revendication 12, une mémoire tampon étant prévue dans laquelle peuvent être enregistrées temporairement des valeurs mesurées continuellement et comprenant en plus un dispositif d'interprétation pour analyser une variabilité des valeurs mesurées.

14. Procédé selon l'une des revendications 12 à 13, la commande de système comprenant un calculateur pour calculer des valeurs de tendance sur la future évolution attendue du paramètre mesuré, notamment pour calculer une dérivation dans le temps des valeurs mesurées, et étant configuré de préférence pour déterminer une dérivation dans le temps des valeurs de tendance.

15. Programme informatique, notamment pour être exécuté sur un dispositif selon l'une des revendications 12 à 14, **caractérisé en ce que** lors d'une exécution sur une machine de traitement de données, il met en oeuvre le procédé selon l'une des revendications 1 à 12.
